# EUROPEAN PATENT SPECIFICATION

(11) **EP 0 473 533 B1**
(45) Date of publication and mention of the grant of the patent: **29.12.1999**
(21) Application number: 91610069.6
(22) Date of filing: 26.08.1991
(51) Int. Cl.: C05F 9/00, C05F 3/00, C12P 5/02

(54) **A method of separating source-sorted organic domestic waste and other organic materials containing undesired foreign bodies**
Verfahren zur Abtrennung von unerwünschten Fremdkörpern aus Haushaltsabfällen verschiedener Herkunft und anderen organischen Materialien
Procédé de séparation des ordures ménagères et d'autres matériaux organiques contenant des corps étrangers

(30) Priority: 27.08.1990 DK 204490
(43) Date of publication of application: 04.03.1992
(73) Proprietor: HERNING KOMMUNALE VAERKER, 7400 Herning (DK); VIGGO FOLMER Ingeniorfirma A/S, 8100 Aarhus C (DK)
(72) Inventor: Lyhne, Poul, DK-7400 Herning (DK)
(74) Representative: Christiansen, Ejvind

(56) References cited:
- FR-A- 888 925
- US-A- 4 522 721

## Description

The present invention concerns a method for the treatment of source-sorted organic domestic waste containing inorganic substances with a view to separate the organic matter from the inorganic substances, thereby obtaining a useful liquid fraction and a solid residual fraction, said treatment consisting in combining the organic starting material with a previously digested material and digesting the combined materials in an anaerobic digestion tank.

Many local authorities have already implemented sorting of domestic waste at source, and more will do so in the years to come, which will result in a relatively acute disposal problem. For Denmark alone, the organic part of the domestic waste can amount to as much as 500,000 tons per year. It is therefore of great importance that the greatest possible amount of the organic part of the source-sorted domestic waste can be separated, while the plant nutritive substances can be used again as a fertilizer on farm land, without this re-use involving any restrictions with respect to the use of the crops from these fields.

The object of the present invention is thus to provide a method by which domestic waste and other organic types of waste which are difficult to handle can be utilized in biogas systems, the objectives of the method being in particular:
- to achieve maximum energy production from the entire amount of the organic material;
- to ensure sanitation enabling non-restrictive re-use of the residual material as a fertilizer in agriculture;
- that the degassed material discharged on the farm land is 100% pure;
- that, technically, the method can be applied to and be integrated in existing common biogas systems, and
- that the method can operate fully automatically without manual service and without working environment problems.

The present invention concerns a method for the treatment of source-sorted organic domestic waste containing inorganic substances with a view to separate the organic matter from the inorganic substances, thereby obtaining a useful liquid fraction and a solid residual fraction, said treatment consisting in combining the organic starting material with a previously digested material and digesting the combined materials in an anaerobic digestion tank, which is characterized in that the organic material, without prior sorting, is fully mixed with the previously digested material, said previously digested material being anaerobically digested farmyard manure having a temperature of 30-60 °C resulting from an anaerobic digestion, and that the mixed materials subsequently are subjected to anaerobic digestion at the same temperature in the digestion tank, following which the biogas formed in the process is collected and the useful liquid traction is separated from the solid residual fraction containing the inorganic substances.

The method of the invention ensures that the useful substances in the domestic waste, including nutritive salts and dissolved organic substances, are present in the aqueous fraction. The method ensures at the same time that undesired foreign bodies, including inorganic substances which cannot be discharged on farm land, remain in the residual fraction.

The method of the invention has the advantage over known methods that when mixing the organic domestic waste with a warm, anaerobically digested material consisting of farmyard manure, the organic components will be decomposed more effectively. This means that the content of organic dry matter in the residual fraction is minimized, while the aqueous fraction, with just an insignificant content of undesired components, can be utilized directly as a fertilizer on farm land.

Thus, the method of the invention serves the double purpose of producing biogas and fertilizers with simultaneous disposal of organic waste whose existence is undesirable e.g. for environmental reasons. It will hereby be possible to include biogas systems which can today only receive farmyard manure and industrial waste which is easy to handle, in the disposal of more difficult types of waste, thereby providing for a more efficient energy and agricultural utilization of these types of waste.

The invention makes it possible to use a much greater portion of the biomass which is deposited or composted today, in the energy supply in connection with e.g. CHP stations, while ensuring that the nutritive substances are recycled to the farm land.

In principle, waste sorted at source can be treated in two different ways, viz. by composting and by anaerobic digestion. The method of the invention is based on anaerobic digestion at a temperature of 30-60°C after addition of an already digested material, which likewise has a temperature of 30-60°C, to ensure effective anaerobic digestion.

It is known that the yield and the reaction rate in digestion processes can be increased by graffing the material to be digested with already digested material. It is likewise known that addition of a warm graff material improves the yield. This art is used e.g. according to the SU Patent Specification 833 935 and DD Patent Specification 278 486.

A process of anaerobically digesting organic wastes under an anaerobic condition in a digestion tank for recovering methane from said organic wastes by the fermentation effect of anaerobic bacteria is disclosed in US-A-4522721 (ISHIDA et al), said process comprising charging organic wastes having a water content of 50-70% and seed digestives alternatively in a strata formation to said digestion tank said organic wastes formed as a layer between alternating layers of said seed digestives and maintaining the interior of said digestion tank under anaerobic conditions without stirring.

According to US-A-4522721 city garbage, agricultural wastes, dehydrated organic sludge etc. may be used as organic waste, and digested feedstock may be recycled for reuse as seed digestives.

It is further mentioned that the gist of the ISHIDA-invention consists in the arrangement of organic waste and seed digestives in strata, that the generation of methane is assumed to take place at the bordering areas of the respective layers, and that comparative experiments show that anaerobic fermentation is impeded when the organic wastes and seed digestives are not arranged in stratas but introduced into the fermentation tank as a mixture.

It has however surprisingly been found that a fully satisfactory fermentation can be obtained by using a starting material consisting of organic material fully mixed with anaerobically digested farmyard manure.

The EP Patent Application 220 647 discloses a method of converting various types of sludge, including liquid manure, by mixing with source-sorted waste and subsequent anaerobic digestion, optionally after basic hydrolysis.

The French Patent Application 2 516 541 describes a method of producing biogas, wherein domestic waste and liquid manure are separately subjected to a pretreatment, following which they are mixed and digested together.

The DE Patent Specification 2 703 571 concerns treatment of domestic waste which is mixed with sludge, water and other liquid waste substances, including organic types of sludge such as liquid manure, in a tank. The mixture is subjected to anaerobic fermentation to provide concentrated sludge for production of paper and cardboard.

Finally, the International Patent Application WO 88/04282 describes anaerobic treatment of conditioned sludge, e.g. originating from domestic waste.

It is common to these last mentioned known methods that prior to the anaerobic digestion undesirable foreign bodies are sorted as well as possible. However, this sorting is expensive, difficult to perform satisfactorily and moreover inexpedient with respect to the working environment. Such initial sorting has so far been considered necessary to obtain a satisfactory procedure.

It has now surprisingly been found that it is possible to omit sorting the foreign bodies first and instead apply the opposite approach; the organic source-sorted domestic waste and optional similar materials are mixed untreated with anaerobically digested farmyard manure which has a temperature of 30-60°C. The mixture is digested in an anaerobic digestion tank likewise at a temperature of 30-60°C. The effective anaerobic digestion results in extensive formation of methane-containing biogas as well as decomposition of the organic material to a liquid fraction, which is then separated in a manner known per se from the undesired foreign bodies.

It is hereby ensured that the useful substances, including nutritive salts and dissolved organic substances, remain in the liquid fraction which can be utilized as a fertilizer on farm land without any subsequent treatment.

The residual fraction containing foreign bodies can be deposited or composted.

The invention will be illustrated more fully by the following example:

### EXAMPLE

The source-sorted domestic waste is received in closed special containers from which it is tipped into a coarse crusher which crushes the material. The crusher is placed on top of a cast mixing tank (88 m³), in which the waste, following crushing, is mixed with raw liquid manure or digested separated liquid manure.

The material is sucked from the mixing tank through a mincer capable of mincing objects to a typical average size of 5.5 x 10 mm. The minced material is passed further on to one of two sanitation tanks, each having a volume of 2.5 m³. The material is heated in the tanks to 70°C by steam, the residence time being at least one hour with stirring.

Two containers are employed, one being used for pumping into the subsequent digestion tank, while the other one is filled and pre-sanitized.

The material is pumped from the deactivation tanks to the digestion tank, to which also separated digested liquid manure from the liquid tank is conveyed. This liquid is added to stabilize the biological process, but also to ensure the optimum digestion temperature (51-55°C). The liquid is obtained by separating digested biomass from an existing biogas system.

The separated liquid manure is pumped to the previously mentioned liquid tank (20 m³), in which it is stored until it is to be used in either digestion tank or mixing tank.

The digestion tank, which has a digestion volume of 210 m³ with a gas volume of 30 m³, is a steel tank with a conical bottom constructed such that it is possible to empty the tank of sediments on the bottom.

Having stayed in the digestion tank for an average of 24 days and nights with full stirring, the material is pumped to a storage tank (2680 m³) in which it is left for final digestion under psychrophilic/mesophilic conditions.

Separation of the long-term digested material in the storage tank is performed every nine months and takes about 10 days. The dry matter content of the resulting residual fraction is 30-40%.

To enable collection of the produced gas from the storage tank, it is covered with a rubber cloth. The produced gas is conveyed to a sludge separation well. Immediately before the well, the gas is united with the gas from the digestion tank.

Part of the sludge of the gas is separated in the sludge separation well.

The total gas amount is then conveyed through buried gas piping to existing gas installation where it is united with the gas from the biogas system.

The gas production amounts to about 200 m³ biogas per ton of sorted domestic waste with a methane content of about 65%.

## Claims

1. A method for the treatment of source-sorted organic domestic waste containing inorganic substances with a view to separate the organic matter from the inorganic substances, thereby obtaining a useful liquid fraction and a solid residual fraction, said treatment consisting in combining the organic starting material with a previously digested material and digesting the combined materials in an anaerobic digestion tank, **characterized** in that the organic material, without prior sorting, is fully mixed with the previously digested material, said previously digested material being anaerobically digested farmyard manure having a temperature of 30-60 °C resulting from an anaerobic digestion, and that the mixed materials subsequently are subjected to anaerobic digestion at the same temperature in the digestion tank, following which the biogas formed in the process is collected and the useful liquid fraction is separated from the solid residual fraction containing the inorganic substances.

2. A method according to claim 1, **characterized** in that the temperature of the previously digested material and the temperature of the subsequent anaerobic digestion of the mixture are both 51-55 °C.

## Patentansprüche

1. Verfahren zum Behandeln von nach Quellen sortiertem, organischem Hausmüll, der anorganische Substanzen enthält, um die organischen Bestandteile von den anorganischen Substanzen zu trennen, so daß eine verwendbare flüssige Fraktion und eine feste Restfraktion erhalten wird, wobei die Behandlung in dem Mischen des organischen Ausgangsmaterials mit einem bereits verfaulten Material und in dem Ausfaulen des gemischten Materials in einem anaeroben Faultank besteht,
**dadurch gekennzeichnet**, daß das organische Material ohne vorherige Sortierung vollständig mit dem bereits verfaulten Material gemischt wird, wobei das bereits verfaulte Material anaerob abgebauter landwirtschaftlicher Dung mit einer Temperatur von 30-60° C ist, der durch anaeroben Abbau erhalten wurde, und daß die gemischten Materialien nachfolgend in dem Faultank bei gleichen Temperatur dem anaeroben Abbau ausgesetzt werden, woran sich ein Sammeln des bei diesem Vorgang entstandenen Biogases und die Separierung der nützlichen flüssigen Fraktion von der festen Restfraktion, die die anorganischen Substanzen enthält, anschließen.

2. Verfahren nach Anspruch 1,
**dadurch gekennzeichnet**, daß die Temperatur des bereits verfaulten Materials und die Temperatur der nachfolgenden anaeroben Verfaulung der Mischung 51-55° C beträgt.

## Revendications

1. Procédé pour le traitement de déchets organiques domestiques triés à la source (〈〈 source sorted organic domestic waste 〉〉) et contenant des substances inorganiques en vue de séparer la matière organique des substances inorganiques et ainsi d'obtention une fraction liquide utile et une fraction solide résiduelle, ledit traitement consistant à combiner la matière organique de départ avec une matière préalablement digérée et de digérer les matières ainsi combinées dans un réservoir de digestion anaérobie, caractérisé en ce que la matière organique, sans triage préalable, est entièrement mélangée avec la matière préalablement digérée, ladite matière préalablement digérée étant du fumier ou lisier de ferme (〈〈 farmyard manure 〉〉) digéré de manière anaérobie et présentant une température de 30 à 60° C résultant d'une digestion anaérobie et caractérisé en ce que les matières mélangées subissent ensuite une digestion anaérobie à la même température dans le réservoir de digestion, puis ensuite le biogas qui s'est formé lors du traitement est collecté et la fraction liquide utile est séparée de la fraction résiduelle solide contenant les substances inorganiques.

2. Procédé selon la revendication 1, caractérisé en ce que la température de la matière préalablement digérée et la température de la digestion anaérobie du mélange qui suit sont toutes deux de l'ordre de 51 à 55° C.
